# EUROPEAN PATENT APPLICATION

(11) **EP 1 306 782 A1**
(43) Date of publication of application: **02.05.2003**
(21) Application number: 01932151.2
(22) Date of filing: 18.05.2001
(51) Int. Cl.: G06F 17/60, A23L 1/00, A23L 1/29

(54) **EXTRAMURAL COOKING CONTROL METHOD AND SYSTEM THEREFOR**

(30) Priority: 22.05.2000 JP 2000149680
(71) Applicant: Daiichi Foods Co., Ltd., Osaka-shi, Osaka 544-0004 (JP)
(72) Inventor: KOMIYA, Hidenori, Ikuno-ku Osaka-shi Osaka 544-0004 (JP); KAWABATA, Kazuki, Ikuno-ku Osaka-shi Osaka 544-0004 (JP)
(74) Representative: Prüfer, Lutz H., Dipl.-Phys.
(86) International application number: JP0104198
(87) International publication number: WO01090979

(57) **Abstract**

It is an object of the invention to provide an out-of-hospital cooking management system using a computer that allows a plurality of different kinds of meals ordered by a plurality of hospitals to be efficiently cooked and accurately provided to patients in the hospitals. By the out-of-hospital cooking management system according to the invention, in response to orders received from a plurality of hospitals (12), a meal prescription for each patient is registered in a meal prescription master file (218), the meal prescription for specified date and time is extracted from the meal prescription master file (218) and eating data (ET2) is produced, and preparation instructions (46) are printed based on the eating data (ET2) (S328). Cooking instructions (56) for the same kind of cooking in bulk is printed based on the eating data (ET2) (S344). Arrangement instructions (60) are printed based on the eating data (ET2) (S362), and delivery instructions (66) for dishes in bulk are printed for each hospital (S368).

## Description

### TECHNICAL FIELD

The present invention relates to an out-of-hospital cooking management method using a computer and a system therefor.

### BACKGROUND ART

Today, meals for inpatients are cooked either in the hospitals, in other words, by in-hospital cooking or outside the hospitals, in other words, by out-of-hospital cooking. The out-of-hospital cooking which was prohibited by law until several years ago is allowed today by deregulation.

In the out-of-hospital cooking today, based on orders received from a hospital, a food service company sequentially carries out preparation (generally also called "pre-cooking"), heating, rapid cooling, and refrigeration at a factory outside the hospital, and the cooked meals are delivered by a refrigerator car to the hospital. Meanwhile, the hospital (or a service company entrusted by the hospital) keeps the delivered meals in the refrigerator in the hospital, re-heats the meals and arranges them on trays in the hospital kitchen.

By employing the out-of-hospital cooking instead of the in-hospital cooking, the kitchen space in the hospital can significantly be reduced, while the space is still necessary for the refrigeration, re-heating and arranging.

In order to do without any kitchen space in the hospital, one method would be to carry out the entire process up to the arrangement of meals on trays outside the hospital. Such out-of-hospital cooking is referred to as "complete out-of-hospital cooking" and the first above-described out-of-hospital cooking is referred to as "incomplete out-of-hospital cooking" to be distinguished from the "complete out-of-hospital cooking."

The complete out-of-hospital cooking may seem possible without much difficulty, but it is not so in practice. This is for the following reasons.

First, meals taken at the hospital vary greatly depending upon the conditions of patients. The principal food, rice is cooked into different states such as normal steamed rice, soft steamed rice, gruel rice such as *zen-gayu, shichibu-gayu, gobu-gayu,* and *sanbu-gayu,* and thin rice water. (The *zen-gayu, shichibu-gayu, gobu-gayu,* and *sanbu-gayu* are cooked with different rice to water ratios of 1: 5, 1: 7, 1: 10, and 1: 20, respectively.) Accompanying dishes can be divided into normal dish, maternity dish, and dish for patients suffering from gestosis, high blood pressure, pancreatic diseases, diabetes, and renal diseases, and infants. Therefore, the food service company must provide such a wide variety of meals. In addition, a meal appropriate for each patient's condition must be delivered without mistakes. Meanwhile, the variety of meals must be provided efficiently and inexpensively.

Furthermore, when orders are taken from more than one hospital, the food service company must take care of management on a hospital-basis in addition to the above patient-based management, and the number of meals to cook increases. Therefore, it was substantially impossible to take orders from a plurality of hospitals and carry out the complete out-of-hospital cooking.

### DISCLOSURE OF THE INVENTION

The present invention is directed to a solution to the above disadvantages, and it is an object of the invention to provide an out-of-hospital cooking management method and a system using a computer that allows a plurality of kinds of meals ordered by a plurality of hospitals to be efficiently cooked and accurately provided to patients in the hospitals.

According to one aspect of the invention, the out-of-hospital cooking management method using a computer includes the steps of, in response to orders from a plurality of hospitals, registering a meal prescription including information related to a patient, a hospital where the patient is hospitalized, and a meal for the patient in a meal prescription master file; searching through the meal prescription master file and outputting cooking instructions for dishes in bulk to be cooked by the same cooking method among a plurality of kinds of dishes; searching through the meal prescription master file and outputting arrangement instructions for meals; and searching through the meal prescription master file and outputting shipping instructions for meals in bulk to be delivered to the same hospital. Herein, the "ordering" may be carried out by transmission of meal prescription data or through telephone or facsimile communication.

According to the cooking instructions, a plurality of kinds of meals may be cooked in bulk on a cooking method basis. Then, according to the shipping instructions, the cooked meals can be delivered in bulk on a hospital basis. Therefore, a plurality of kinds of meals ordered by a plurality of hospitals can be efficiently cooked and accurately delivered to patients in the hospitals.

The step of outputting the cooking instructions preferably includes the step of outputting principal food cooking instructions for principal food portions in bulk to be cooked by the same method.

According to the principal food cooking instructions, principal food portions of the same kind can be cooked in bulk.

Alternatively, the step of outputting the cooking instructions includes the step of outputting preparation instructions for accompanying dishes in bulk to be prepared by the same preparation method.

According to the preparation instructions, preparations for accompanying dishes can be carried out in bulk on a preparation kind basis.

Alternatively, the step of outputting the cooking instructions includes the step of outputting cooking instructions for accompanying dishes in bulk to be cooked by the same cooking method.

According to the preparation instructions, accompanying dishes can be cooked in bulk on a cooking method basis.

The out-of-hospital cooking management method preferably further includes the step of extracting meal prescriptions for the same eating date and time from the meal prescription master file and producing an eating data file.

The above cooking, arrangement, and shipping instructions can be output more quickly by searching through the eating data file and indirectly searching through the meal prescription master file than directly searching through the meal prescription file.

The step of outputting the principal food cooking instructions more preferably includes the step of searching through the eating data file and calculating the number of principal food portions of the same kind.

Alternatively, the step of outputting the preparation instructions includes the step of searching through the eating data file and calculating the number of accompanying dishes of the same kind, searching through a menu master file that stores the contents of accompanying dishes and specifying the contents of each accompanying dish and searching through a cooking master file that stores preparation methods and presenting a preparation method for each of the accompanying dishes whose contents are specified.

Alternatively, the step of outputting the cooking instructions includes the steps of searching through the eating data file and calculating the number of accompanying dishes of the same kind, searching through a menu master file that stores the contents of accompanying dishes, and specifying the contents of each accompanying dish, and searching through a cooking master file that stores cooking methods, and presenting a cooking method for each of the accompanying dishes whose contents are specified.

The out-of-hospital cooking management method preferably further includes the steps of printing a meal card having at least the name of a patient, the step of outputting the shipping instructions includes the step of printing a cart list having at least the names of patients to receive a plurality of meals to be loaded on each cart.

When a meal card is set corresponding to a meal at the time of meal arrangement, meals can accurately be loaded to each cart based on a cart list.

The step of outputting the shipping instructions more preferably further includes the step of printing delivery instructions having at least the name of a hospital to which the cart is to be delivered.

According to the delivery instructions, carts can accurately be delivered to the hospitals.

According to another aspect of the invention, an out-of-hospital cooking management system using a computer includes a meal prescription master file, meal prescription registering means, cooking instruction means, arrangement instruction means, and shipping instruction means. The prescription master file stores a meal prescription including information related to a patient, a hospital where the patient is hospitalized, and a meal for the patient. The meal prescription registering means registers the meal prescription in the meal prescription master file in response to orders received from a plurality of hospitals. The cooking instruction means searches through the meal prescription master file and outputs cooking instructions for dishes in bulk to be cooked by the same cooking method among a plurality of kinds of dishes. The arrangement instruction means searches through the meal prescription master file and outputs arrangement instructions for meals. The shipping instruction means searches through the meal prescription master file and outputs shipping instructions for meals in bulk to be delivered to the same hospital.

According to the cooking instructions, a plurality of kinds of dishes can be cooked in bulk, and according to the shipping instructions, the cooked meals can be delivered in bulk on a hospital-basis. Therefore, a plurality of kinds of meals ordered by a plurality of hospitals can be efficiently cooked and accurately provided to patients in the hospitals.

The cooking instruction means preferably includes principal food cooking instruction means for outputting principal food cooking instructions for principal food portions in bulk to be cooked by the same cooking method.

According to the principal food cooking instructions, principal food portions may be cooked in bulk on a principal food kind basis.

The cooking instruction means preferably includes preparation instruction means for outputting preparation instructions for accompanying dishes in bulk to be prepared by the same preparation method.

According to the preparation instructions, preparations for accompanying dishes can be carried out in bulk on a method basis.

The cooking instruction means preferably further includes cooking instruction means for outputting cooking instructions for accompanying dishes in bulk to be cooked by the same cooking method.

According to the cooking instructions, accompanying dishes can be cooked in bulk on a method basis.

The out-of-hospital cooking management system preferably further includes means for extracting meal prescriptions for the same eating date and time from the meal prescription master file and producing an eating data file.

The above cooking, arrangement, and shipping instructions can be output more quickly by searching through the eating data file and indirectly searching through the meal prescription master file than directly searching through the meal prescription file.

The principal food cooking instruction means preferably includes means for searching through the eating data file and calculating the number of principal food portions of the same kind.

The preparation instruction means preferably includes a menu master file that stores the contents of accompanying dishes, a cooking master file that stores preparation methods, means for searching through the eating data file and calculating the number of accompanying dishes of the same kind, means for searching through the menu master file and specifying the contents of each accompanying dish, and means for searching through the cooking master file and presenting a preparation method for each of the accompanying dishes whose contents are specified.

The cooking instruction means preferably includes a menu master file that stores the contents of accompanying dishes, a cooking master file that stores cooking methods, means for searching through the eating data file and calculating the number of accompanying dishes of the same kind, means for searching through the menu master file and specifying the contents of each accompanying dish, and means for searching through the cooking master file and presenting a cooking method for each of the accompanying dishes whose contents are specified.

The arrangement instruction means preferably includes means for printing a name card having at least the name of a patient. The shipping instruction means includes means for printing a cart list having at least the names of patients to receive a plurality of meals to be loaded on each cart.

When a meal card is set corresponding to a meal at the time of arrangement, the meal can accurately be loaded onto each cart according to the cart list.

The shipping instruction means preferably includes means for printing delivery instructions including at least the name of a hospital to which the cart is to be delivered.

According to the delivery instructions, carts can accurately be delivered to the hospitals.

### BRIEF DESCRIPTION OF THE DRAWINGS

Fig. 1 is a diagram showing a general idea about how out-of-hospital cooking is carried out;
Fig. 2 is a block diagram of the general configuration of an out-of-hospital cooking management system provided at the food service company in Fig. 1;
Fig. 3 is a block diagram of the general configuration of a meal ordering terminal provided at each of the hospitals in Fig 1;
Fig. 4 is a flow chart for use in illustration of the operation of the out-of-hospital cooking management system in Fig. 2 and the meal ordering terminal shown in Fig. 3;
Fig. 5 shows the record structure of a meal prescription transaction file transmitted from a hospital to the food service company in Fig. 4;
Fig. 6 is a table for use in illustration of data kinds in the meal prescription transaction file in Fig. 5;
Fig. 7 is a table showing an example of the meal prescription master file in Fig. 2;
Fig. 8 is a table showing an example of the meal prescription transaction file transmitted from a hospital to the food service company in Fig. 4;
Fig. 9 is a table showing an example of the meal prescription master file in Fig. 7 after the file is updated by the meal prescription transaction file shown in Fig. 8;
Fig. 10 is a table showing an example of the eating data shown in Fig. 4;
Fig. 11 is a flow chart continued from Fig. 4;
Fig. 12 is a table showing an example of the eating data shown in Fig. 11;
Fig. 13 is the principal food kind based dish number table obtained in step S300 shown in Fig. 11;
Fig. 14 is a table showing the principal food amount table shown in Fig. 11;
Fig. 15 is the principal food kind based cooking amount table obtained in step S302 shown in Fig. 11;
Fig. 16 is a table showing an example of the principal food cooking instructions shown in Fig. 11;
Fig. 17 is the dish kind based dish number table obtained in step S328 shown in Fig. 11;
Fig. 18 is a table showing the structure of the menu master file shown in Fig. 11;
Fig. 19 is a meal combination table obtained in S322 shown in Fig. 11;
Fig. 20 is a table showing the structure of the cooking master file shown in Fig. 11;
Fig. 21 is a table showing an example of an ingredient combination table obtained in step S324 shown in Fig. 11;
Fig. 22 is a table showing another example of the ingredient combination table obtained in step S324 shown in Fig. 11;
Fig. 23 shows an example of the preparation instructions shown in Fig. 11;
Fig. 24 is a table showing an example of the eating data shown in Fig. 11;
Fig. 25 is a table showing an example of a state-based dish number table obtained in step S340 shown in Fig. 11;
Fig. 26 is a table showing an example of the dish kind based menu table for kitchen shown in Fig. 11;
Fig. 27 shows an example of the cooking instructions shown in Fig. 11;
Fig. 28 shows another example of the cooking instructions shown in Fig. 11;
Fig. 29 shows another example of the cooking instructions shown in Fig. 11;
Fig. 30 is a flow chart continued from Fig. 11;
Fig. 31 shows an example of the arrangement instructions shown in Fig. 30;
Fig. 32 shows another example of the arrangement instructions shown in Fig. 30;
Fig. 33 shows an example of the meal card shown in Fig. 30;
Fig. 34 shows an example of the cart list shown in Fig. 30; and
Fig. 35 shows an example of the delivery instructions shown in Fig. 30.

### BEST MODE FOR CARRYING OUT THE INVENTION

Embodiments of the invention will now be described in conjunction with the accompanying drawings. Note that the same or corresponding portions will be denoted by the same reference numerals and will not be described.

### General Idea About Complete Out-of-hospital Cooking

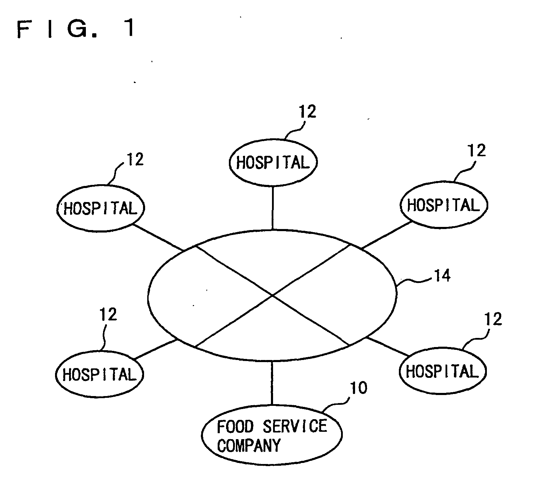

Fig. 1 is a diagram showing a general idea about how complete out-of-hospital cooking is carried out. Referring to Fig. 1, a food service company 10 receives orders from a plurality of hospitals 12 for meals to be provided to their inpatients through the Internet 14. The food service company 10 cooks the ordered meals and delivers the meals arranged on trays to the hospitals 12. The hospitals 12 provide their inpatients with the delivered meals.

### Hardware Configuration of Out-of-hospital Cooking Management System

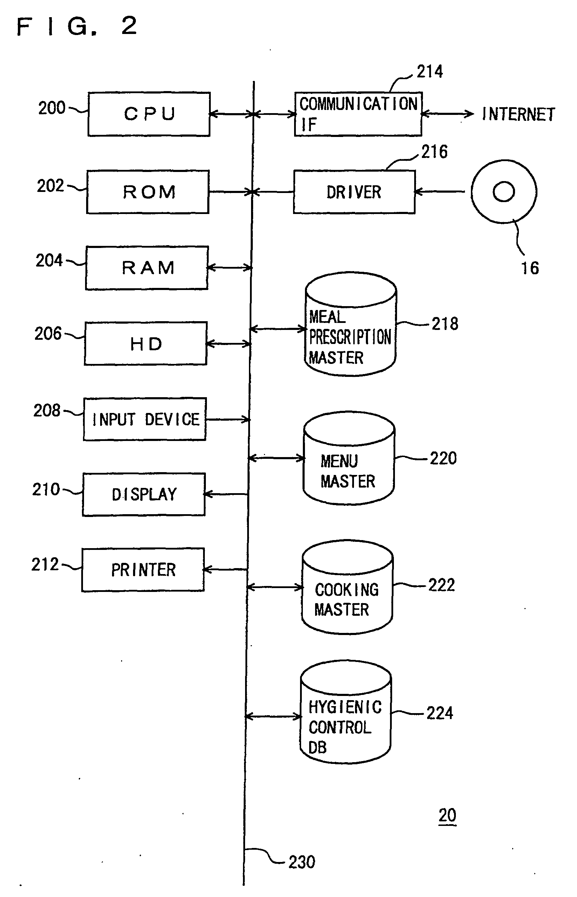

Fig. 2 is a block diagram of the hardware configuration of an out-of-hospital cooking management system 20 provided at the food service company 10. Referring to Fig. 2, the out-of-hospital cooking management system 20 includes a Central Processing Unit (CPU) 200, a Read Only Memory (ROM) 202, a Random Access Memory (RAM) 204, a hard disc 206, an input device 208, a display 210, a printer 212, a communication interface 214, a CD-ROM driver 216, a meal prescription master file 218, a menu master file 220, a cooking master file 222, a hygienic control database 224, and a system bus 230 that connects them with each other and transfers a control signal and a data signal to them. A CD-ROM 16 recorded with an out-of-hospital cooking management program that will be described is set at the driver 216 for installation into a computer, and then the computer serves as the out-of-hospital cooking management system 20.

### Hardware Configuration of Meal Ordering Terminal

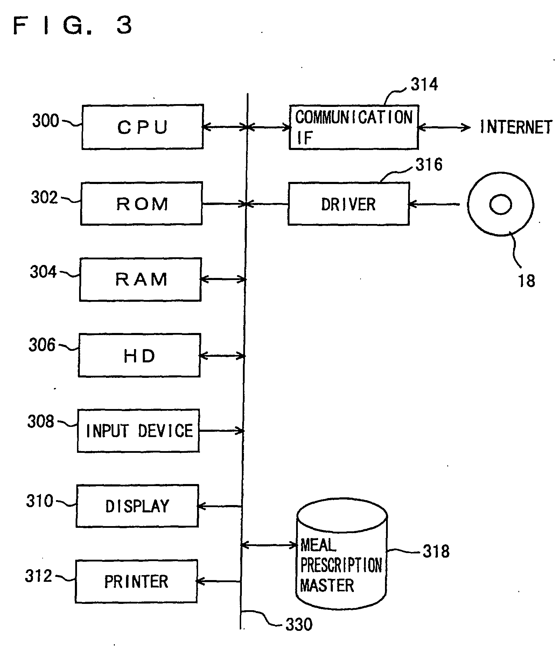

Fig. 3 is a block diagram of the hardware configuration of a meal ordering terminal 30 provided at each of the hospitals 12. Referring to Fig. 3, the meal ordering terminal 30 includes a CPU 300, a ROM 302, a RAM 304, a hard disc 306, an input device 308, a display 310, a printer 312, a communication interface 314, a CD-ROM driver 316, a meal prescription master file 318, and a system bus 330 that connects them with each other and transfers a control signal and a data signal to them. A CD-ROM 18 recorded with a meal ordering program that will be described is set at the driver 316 for installation into a computer, and then the computer serves as the meal ordering terminal 30.

### Operation of Meal Ordering Terminal

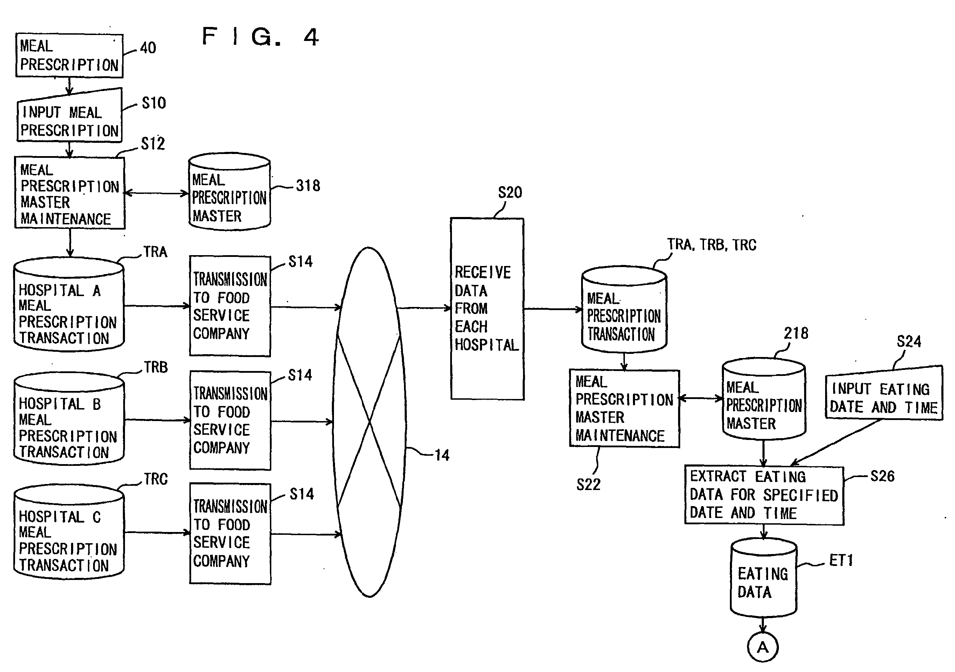

The operation of the meal ordering terminal 30 shown in Fig. 3 will now be described in conjunction with Fig. 4. The above-described meal ordering program includes steps S10 to S14 in Fig. 4.

Based on the hospital rules and regulations, a doctor in charge of a patient produces a meal prescription 40 (a written paper document, etc.). The meal prescription 40 has the name of the hospital, a ward number, a room number, the name of the patient, and written information related to the diet of the patient including eating date and time, the kind of principal food, the kind and state of dish.

The operator inputs the information written in the meal prescription 40 using the input device 308 of the meal ordering terminal 30 shown in Fig. 3. The input device 308 such as a keyboard and a mouse device inputs the information in the meal prescription 40 into a computer in response to the operation of the operator (S10).

The CPU 300 registers the information in the input meal prescription 40 in the meal prescription master file 318. In this way, the meal prescription master file 318 is updated.

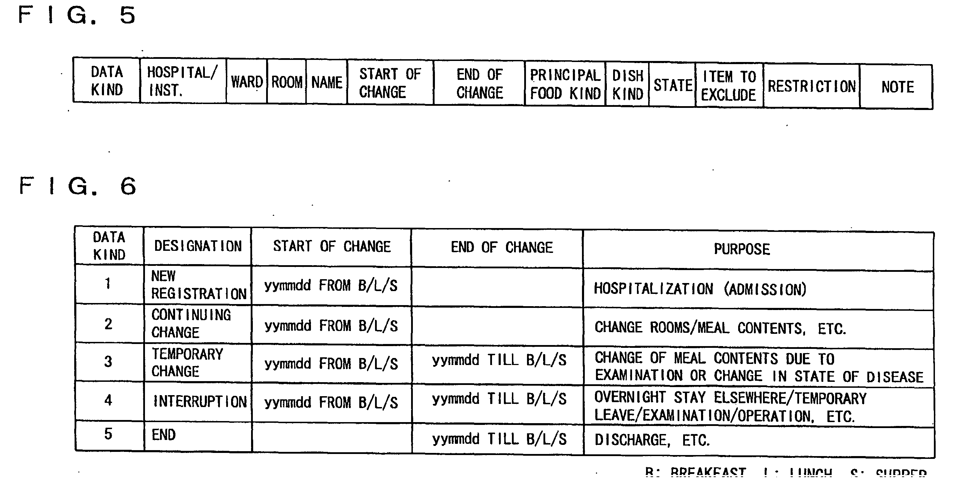

Fig. 5 shows the record structure of the meal prescription master file 318. Referring to Fig. 5, each record includes "data kind," "hospital/institution name," "ward number," "room number," "patient's name," "start of change," "end of change," "principal food kind," "dish kind," "state," "item to be excluded," "restriction," and "note" sections.

As shown in Fig. 6, the "data kind" includes "1" to "5." The numeral "1" represents "new registration" and is used when a new patient is hospitalized. The numeral "2" represents "continuing change" and is used when a patient changes rooms, or the contents of meals are changed. The numeral "3" represents "temporary change" and is used when the contents of meals are changed because of necessary examination or changes in the conditions of disease. The numeral "4" represents "interruption" and is used when meals are not necessary because the patient stays overnight elsewhere or temporarily leaves for outing, examination, or operation. The numeral "5" represents "end" and is used when for example the patient leaves the hospital.

In the column of "start of change," the date to start the meal is recorded, and in the "end of change" column, the last date when the meal is needed is recorded. More specifically, "date," and any of "breakfast," "lunch," and "supper" are entered.

The state of cooked rice is entered in the column of "principal food kind." More specifically, the normal state cooked rice is marked "steamed rice," and then depending upon the condition of disease, "soft steamed rice," "*zen-gayu*," *"shichibu-gayu," "gobu-gayu,"* "*sanbu-gayu*," or "rice water" is entered.

In the column of "dish kind," the kind of accompanying dishes is entered. Normal state dish is marked "normal," and then depending upon the condition of the patient, "maternity," "gestosis," "high blood pressure," "pancreatic," "diabetes," "renal," or "infant," is entered.

In the column of "state," the state of accompanying dishes is entered. For a normal state, the space is left blank, while otherwise it is filled with "cut," "mixer," and the like depending on the condition of the patient.

In the column of "excluded item," items to be excluded (items the patient is allergic to, etc.) are entered. For a patient without any such problem, the space is left blank, while otherwise "no egg," "no milk," "no fish," or the like is entered depending upon the condition of the patient.

In the column of "restriction," restriction on the intake amount of salt, sugar, calorie, etc. is entered. For a patient without such restriction, the space is left blank, while otherwise "salt: not more than 7.0g," "sugar: not more than 15g," "1800 Kcal or less" or the like is entered.

In the column of "note," special information that does not correspond to any of the items above is entered. For a patient without any such information, the space is left blank, while otherwise "fast" or the like is entered depending upon the condition of the patient.

As described above, the meal prescription master file 318 at the meal ordering terminal 30 stores meal prescription data for each inpatient.

Note that when the contents of meal for a certain patient change and the change is previously known, meal prescription data including the change as a whole is desirably input. When there is for example a known meal schedule that goes like "fast," from supper on the previous day of operation, "*zen-gayu*" for supper after the operation, and *"gobu-gayu"* from supper on the day after the operation, the meal schedule as a whole is input in order to reduce errors such as omission.

The CPU 300 then produces a meal prescription transaction file TRA based on the meal prescription data read out from the meal prescription master file 318 every time the input device 308 inputs information in the meal prescription 40 in response to the operation by the operator (S12).

The CPU 300 then transmits the meal prescription transaction file TRA to the food service company 10 by the communication interface 314 through the Internet 14 (S14).

Similarly to the hospital A as described above, a meal prescription transaction file TRB is produced in the hospital B, and the file is transmitted to the food service company 10 through the Internet 14. In the hospital C, a meal prescription transaction file TRC is produced and transmitted to the food service company 10 through the Internet 14. The record structure of the meal prescription transaction files TRA to TRC is the same as that of the meal prescription transaction master file 318 shown in Fig. 5.

### Operation of Out-of-hospital Cooking Management System

The operation of an out-of-hospital cooking management system 20 shown in Fig. 2 will now be described in conjunction with Figs. 4, 11, and 30. The out-of-hospital cooking management program includes the steps S20 to S370 in Figs. 4, 11, and 30.

A CPU 200 in the out-of-hospital cooking management system 20 shown in Fig. 2 receives meal prescription transaction files TRA, TRB, and TRC transmitted from hospitals through a communication interface 214 (S20).

The CPU 200 then registers the received meal prescription transaction files TRA, TRB, and TRC in a meal prescription master file 218, and thus carries out maintenance to the meal prescription master file 218 (S22).

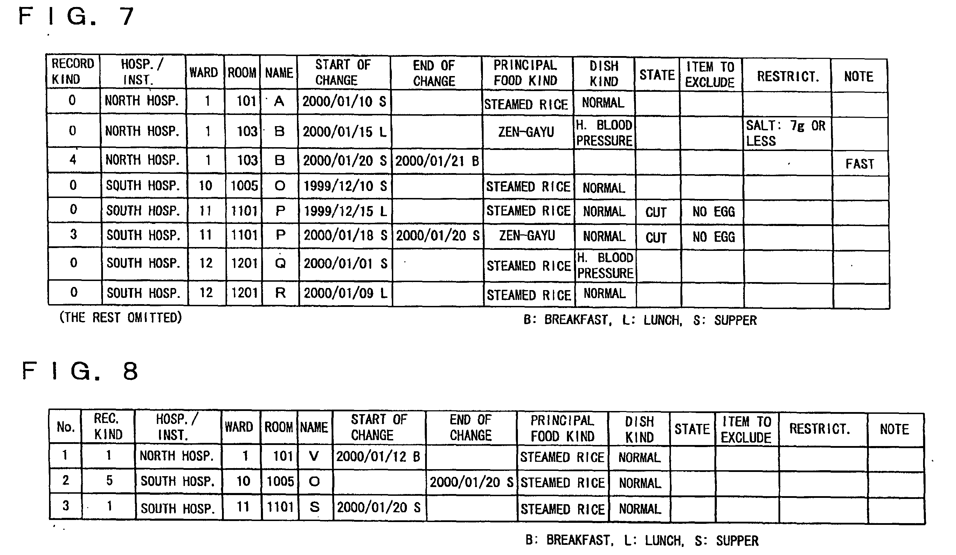

The meal prescription master file 218 stores, for example, meal prescription data as shown in Fig. 7. In this example, the registration for patient A goes like "patient A in room 101, ward 1, North hospital needs steamed rice as principal food and a normal dish as the dish kind (the kind of accompanying dishes) starting from supper January 10, 2000."

Assume now that the meal prescription data as shown in Fig. 7 is registered in the meal prescription master file 218, and the meal prescription transaction as shown in Fig. 8 is received. In this example, the registration for patient V goes like "patient V admitted to room 101, ward 1, North hospital needs steamed rice as principal food and a normal dish as the dish kind starting from breakfast January 21, 2000."

In this meal prescription transaction file, the CPU 200 newly registers record No. 1 in the meal prescription master file 218, adds record No. 2 to the meal prescription master file 218, and newly registers record No. 3 in the meal prescription master file 218 (S22). In this way, the meal prescription master file 218 is updated.

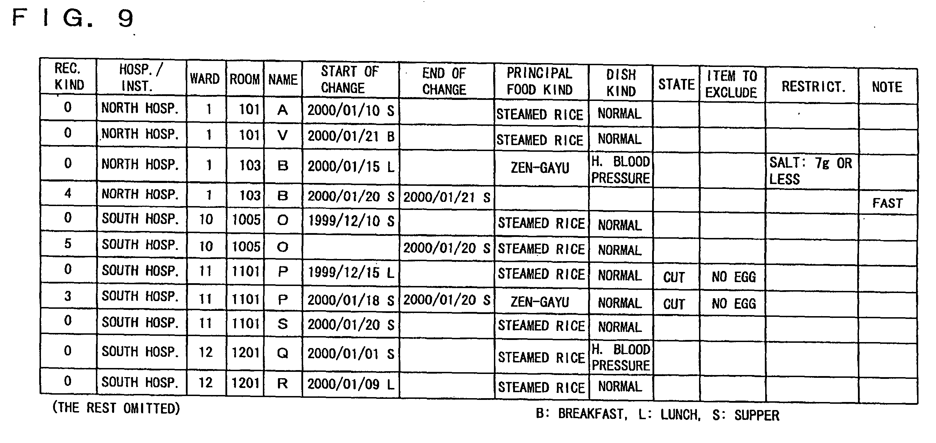

As a result, the contents of the meal prescription master file 218 are as shown in Fig. 9. In the updated meal prescription master file 218, the data of the meal prescription transaction file shown in Fig. 8 is added to the original data of the meal prescription master file 218 in Fig. 7.

Then, the operator inputs desired eating date and time using the input device 208. The input device 208 inputs the desired eating date and time in response to the operation by the operator (S24).

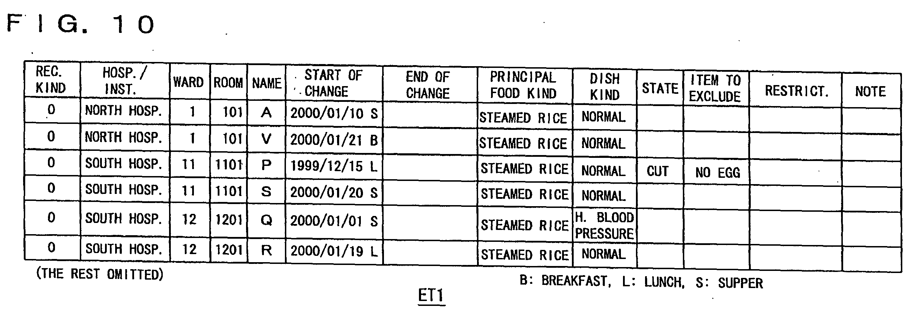

The CPU 200 then extracts eating data ET1 for the date and time of interest from the meal prescription master file 218 in response to the input eating date and time (S26). When for example "lunch on January 21, 2000" is specified, the eating data ET1 as shown in Fig. 10 is extracted. In this example, meal prescription data for patients A, V, P, S, Q, and R is extracted. Patient B is supposed to fast until after supper on January 21, 2000 and therefore the meal prescription data for patient B is not extracted. For patient O, supper on January 20, 2000 is the last meal, and therefore the meal prescription data for patient O is not extracted. Patient P is supposed to have "*zen-gayu*" for supper on and before January 20, 2000, and the original "steamed rice" is to be served from January 21, 2000 for breakfast. Therefore, the extracted prescribed meal data for patient P is "steamed rice."

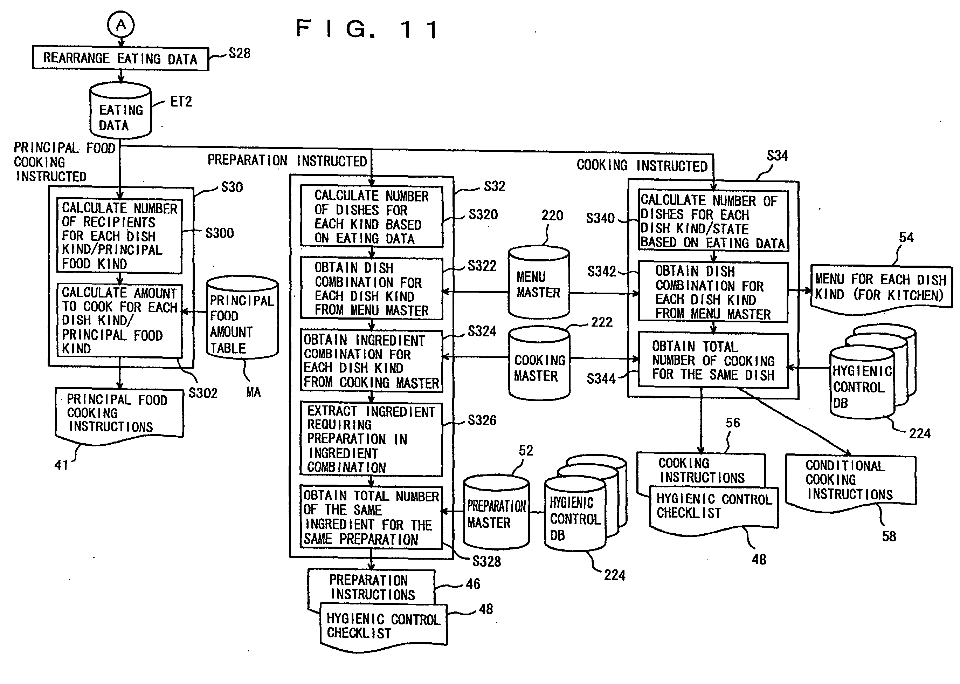

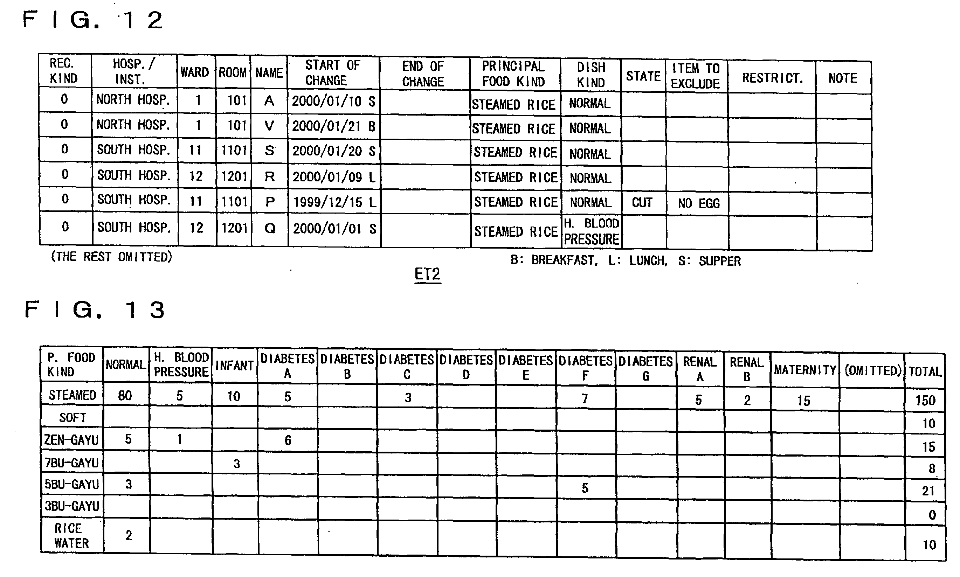

Then, referring to Fig. 11, the CPU 200 rearranges the extracted eating data ET1 in the order of dish kind, principal food kind, restriction, hospital name, ward number, room number, and patient's name (S28). As a result, eating data ET2 as shown in Fig. 12 is obtained. In this example, patient Q who needs dish for "high blood pressure" is moved downward. The eating data for patient P who needs "cut" is moved to the position immediately thereabove.

The CPU 200 then outputs cooking instructions based on the eating data ET2. More specifically, cooking instructions for principal food are output (S30), then preparation instructions for accompanying dishes (how to process ingredients, etc.) are output (S32), and then cooking instructions for accompanying dishes (heating the ingredients after preparation, etc.) are output (S34).

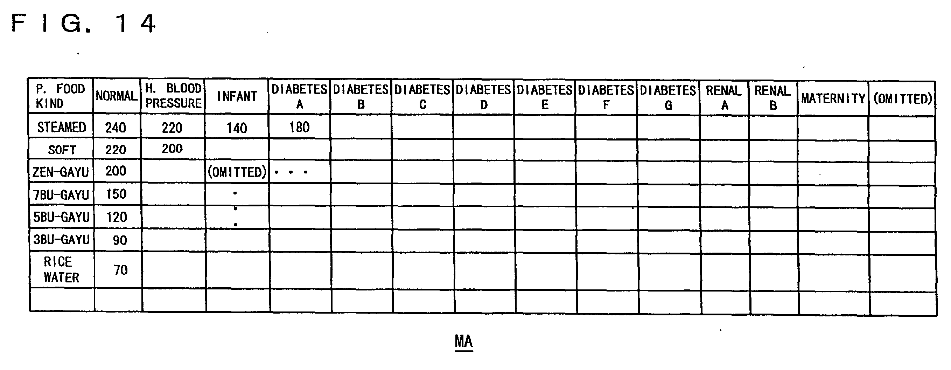

In order to output the cooking instructions for principal food, the CPU 200 calculates the number of recipients for each principal food kind on a dish kind basis (S300). As a result, a dish number table on a principal food kind basis as shown in Fig. 13 is produced and stored in the RAM 204. In this example, the number of recipients who need "normal dish" is 90 in total, of which 80 recipients need "steamed rice," five need "*zen-gayu*," three need "*gobu-gayu*," and two need "rice water." Meanwhile, the number of recipients who need "steamed rice" is 150 in total, of which 80 need "normal dishes," five need "dishes for diabetes A," ten need "infant dishes," three need "dishes for diabetes C," seven need "dishes for diabetes F," five need "dishes for renal disease A," two need " dishes for renal disease B," and 15 need "maternity dishes."

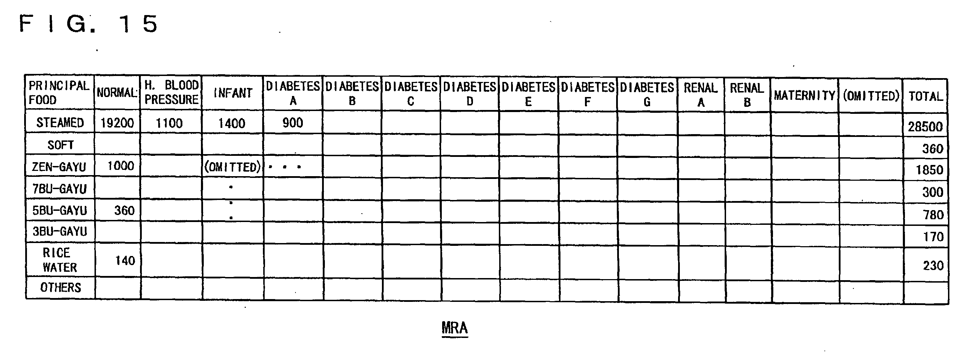

The CPU 200 then calculates the necessary amount to cook for each kind of principal food on a dish kind basis from the principal food kind based dish number table MR by referring to the principal food amount table MA as shown in Fig. 14 (S302). As a result, a principal food kind based cooking amount table MRA as shown in Fig. 15 is obtained.

In the principal food amount table MA shown in Fig. 14, the amount of "steamed rice" for "normal dish" is 240 g per person, the amount of "soft steamed rice" for "normal dish" is 220 g per person, the amount of "*zen-gayu*" for "normal dish" is 200 g per person, the amount of *"shichibu-gayu"* for "*normal* dish" is 150 g, the amount of *"gobu-gayu"* for "normal dish" is 120 g per person, the amount of "*sanbu-gayu*" for "normal dish" is 90 g per person, and the amount of "rice water" for "normal dish" is 70 g. These amounts are predetermined based on the kinds of principal food and the dish kinds.

When the numbers in the spaces in the principal food kind based dish number table MR shown in Fig. 13 are multiplied by the numbers in the corresponding spaces in the principal food amount table MA shown in Fig. 14, the corresponding values in the principal food kind based cooking amount table MRA in Fig. 15 can be produced. For example, the necessary amount of "steamed rice" for "normal dishes" is 19200 g(82× 240 g/person), and the necessary amount of "steamed rice" in total is 28500 g.

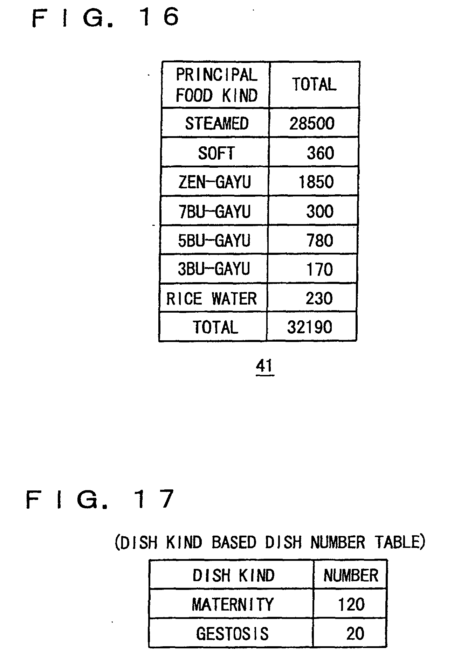

The printer 212 then prints principal food cooking instructions 41 as shown in Fig. 16 (S302). It is known from the principal food instructions 41 that for example 28500 g "steamed rice" is necessary. The worker in charge of cooking the principal food cooks the necessary amount of "steamed rice" by referring to the principal food cooking instructions 41. In order to provide 28500 g "steamed rice" for example, 11400 g (28500g/2.5) rice is cooked.

In order to give preparation instructions for accompanying dishes, the CPU 200 calculates the necessary number of dishes for each dish kind based on the eating data ET2 (S320). Then, for example, the dish kind based dish number table as shown in Fig. 17 is obtained. In this example, the necessary number of "maternity dishes" is 120, and the necessary number of "dishes for gestosis" is 20.

The CPU 200 then searches through the menu master file 220 and specifies the contents of dishes for each dish kind (S322).

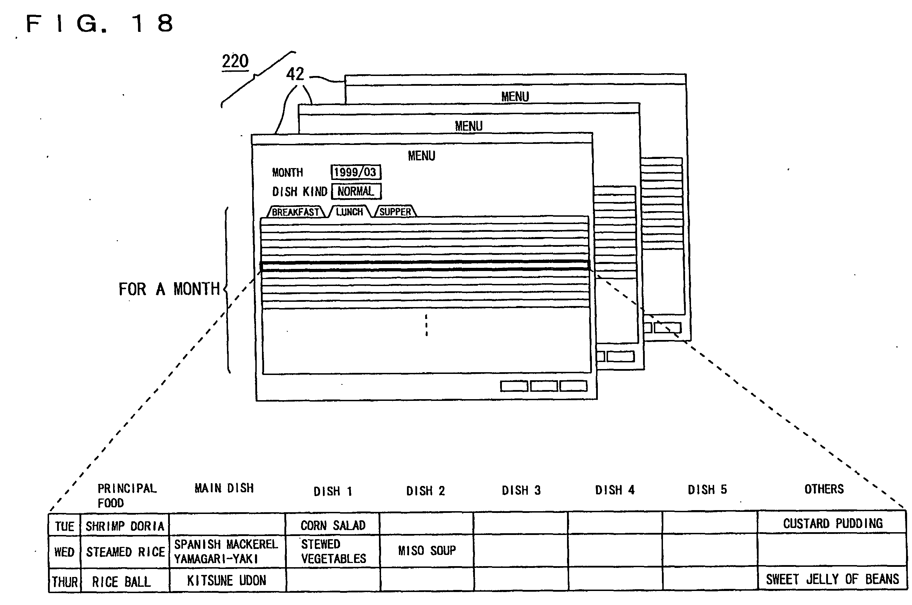

The menu master file 220 stores a plurality of menu lists 42 corresponding to a plurality of dish kinds as shown in Fig. 18. In each menu list 42, menus for one month for a certain dish kind are stored. Each menu list 42 also has pages such as "breakfast," "lunch," and "supper." For example, if the tab 44 in "lunch" is clicked, the page for "lunch" appears on the display 210. This page includes the contents of lunch for one month for a certain dish kind. According to the menu list 42 in the front row in Fig. 18, the lunch on March 10, 1999, Wednesday includes "steamed rice" as the principal food, "Spanish mackerel *yamagariyaki,*" "stewed vegetables," "miso soup," and "custard pudding" as the accompanying dishes.

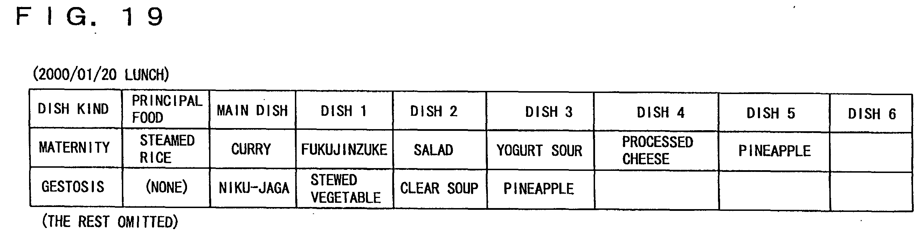

As a result of step S322, a meal combination table as shown in Fig. 19 for example is obtained. In this example, the maternity meal for lunch on January 21, 2000 includes "steamed rice" as the principal food, and "curry," *"fukujinzuke"* (pickled vegetables), "salad," "yogurt sour," "processed cheese," and "pineapple" as the accompanying dishes.

The CPU 200 then searches through the cooking master file 222 and specifies the contents of ingredients for each dish (S324).

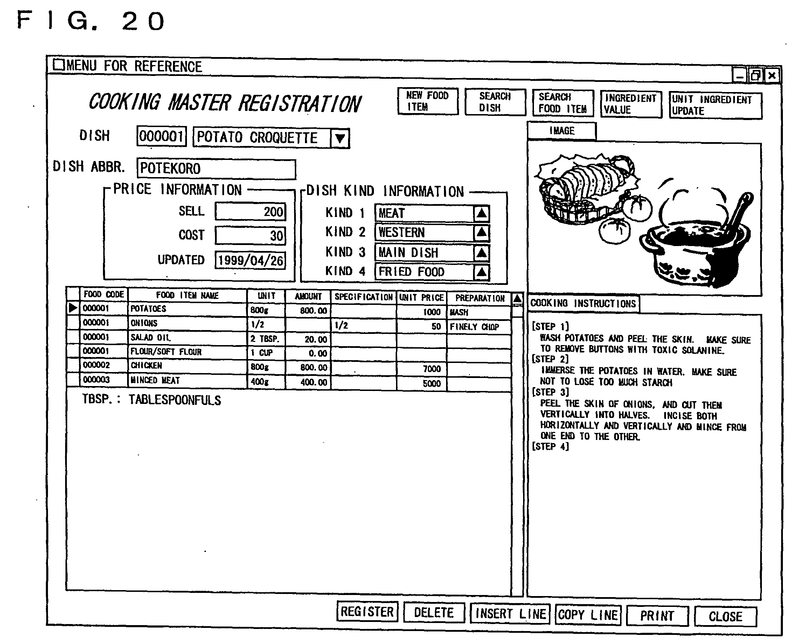

As shown in Fig. 20, ingredients for each dish, and how to prepare and cook them are pre-registered in the cooking master file 222.

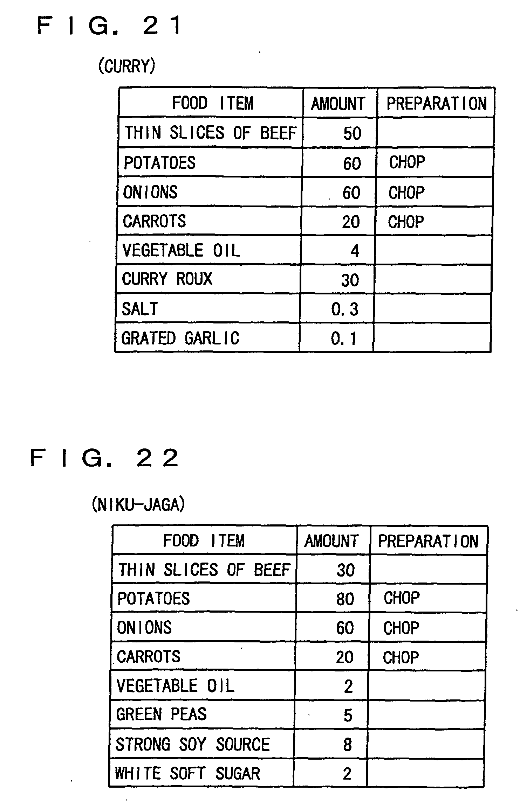

As a result in step S324 as described above, for the "curry," an ingredient combination table as shown in Fig. 21 is obtained. From the ingredient combination table, in order to cook the "curry," "slices of beef," "curry roux," etc. are necessary, while "potatoes," "onions," and "carrots" must be chopped.

Meanwhile, for "*niku-jaga*" (braised beef and potatoes), an ingredient combination table as shown in Fig. 22 is obtained. As can be seen from this ingredient combination table, in order to cook the *"niku-jaga,"* "slices of beef," "green peas," etc. are necessary while "potatoes," "onions," and "carrots" must be chopped.

The CPU 200 extracts ingredients that need preparation from the ingredient combination table obtained in the above step S324 (S326). In the example, "potatoes," "onions," and "carrots" are extracted.

The CPU 200 then sums up and obtains the total necessary amount of the same ingredient items to be subjected to the same preparation (S328).

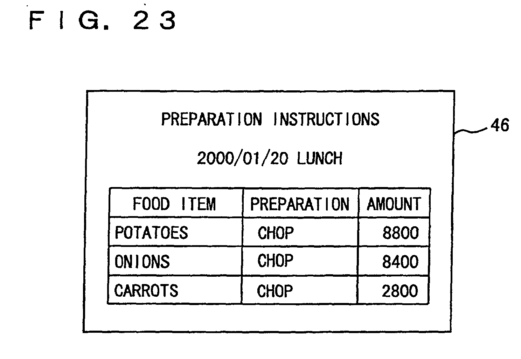

The printer 212 then prints preparation instructions 46 as shown in Fig. 23. In this example, 8800 g (60 g x 120 + 80 g x 20) potatoes, 8400 g (60 g × 120 + 60 g × 20) onions, and 2800 g (20 g × 120 + 20 g x 20) carrots must be chopped altogether. According to the preparation instructions 46, the worker in charge of preparation prepares just the necessary amount of each ingredient.

In the above preparation step, hygienic control is desirably carried out according to HACCP (Hazard Analysis Critical Control Point). In the hygienic control database 224, check items necessary for hygienic control based on the kind of preparation are pre-registered. The CPU 200 extracts check items corresponding to preparation registered in the cooking master file 222 from the hygienic control data base 224 and produces a preparation master file 52. The printer 212 also prints a hygienic control checklist 48 corresponding to the preparation instructions 46.

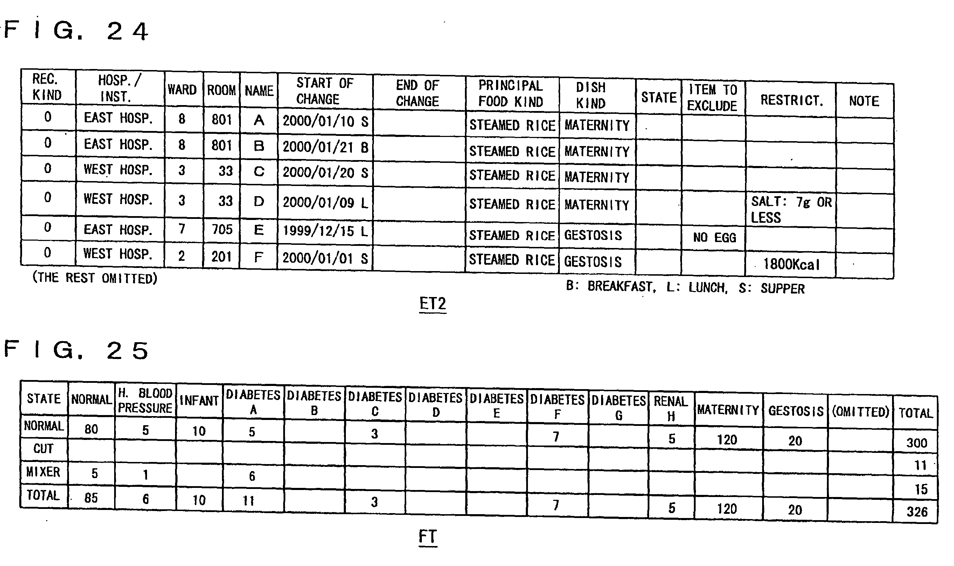

Further in order to instruct how to cook accompanying dishes, the CPU 200 searches through the eating data ET2 and calculates the number of dishes for each dish kind for each state (S340). When for example the eating data ET2 as shown in Fig. 24 is obtained, a state based dish number table FT as shown in Fig. 25 is obtained as a result of step S340 described above. In this example, the necessary number of "normal state" is 300 in total, of which the necessary number of "normal dish" for example is 80. Note that eating data including an item to be excluded, restriction, or a note is not included in the calculation to produce the state based dish number table FT.

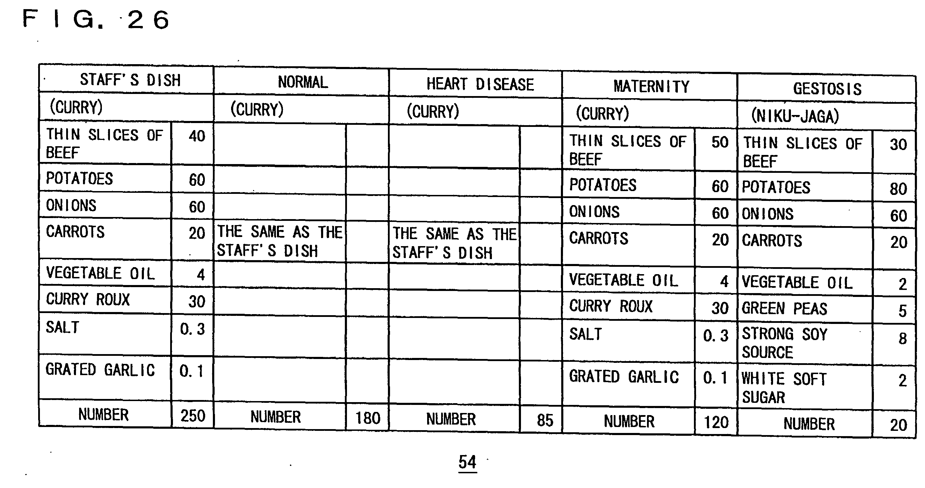

Similarly to the step S322 as described above, the CPU 200 then searches through the menu master file 220, and specifies the contents of cooking for each dish kind (S342). The printer 212 prints a dish kind based menu list for kitchen 54 as shown in Fig. 26 based on the obtained cooking data. The dish kind based menu list 54 also includes "dishes for staffs." In this way, meals are provided not only to patients but sometimes also to hospital staffs.

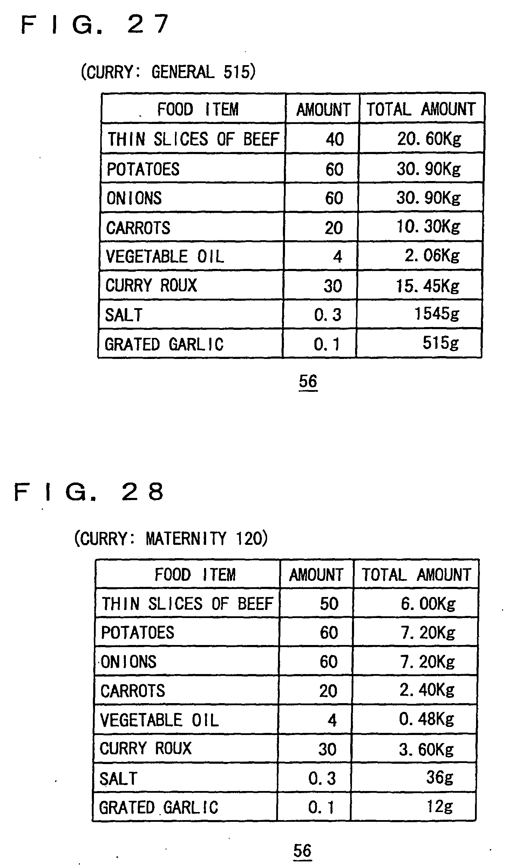

The CPU 200 then searches through the cooking master file 222 and specifies how to cook the dishes specified in step S342 described above, and sums up dishes to be cooked by the same cooking method on a method-basis (S344). Based on the result, the printer 212 prints cooking instructions 56. In the menu shown in Fig. 26, "curry" for "staffs," "normal," and "heart disease" can be cooked by the same method, and therefore the cooking instructions 56 as shown in Fig. 27 are output. The worker in charge of cooking is to cook "curry" for 515 portions in total at a time according to the cooking instructions 56.

For the "curry" for "maternity dish," the cooking instructions 56 as shown in Fig. 28 are output. The worker in charge of cooking is to cook the "curry" for "maternity dish" for 120 portions at a time according to the cooking instructions 56.

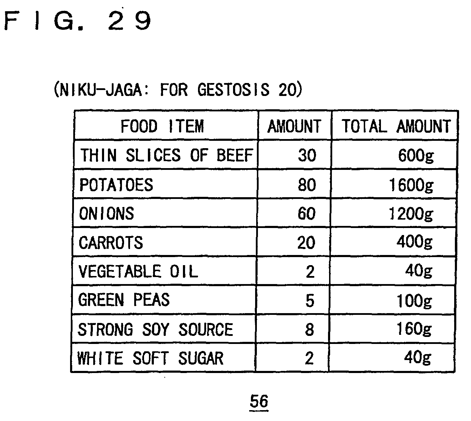

For the *niku-jaga* for "gestosis," the cooking instructions 56 as shown in Fig. 29 are output. The worker in charge of cooking is to cook *niku-jaga* for gestosis for 20 portions at a time according to the cooking instructions 56.

Note that for the eating data including an item to exclude, restriction and a note which is excluded from the process in step S340, the printer 212 separately prints conditional cooking instructions 58. The worker in charge of cooking is to cook dishes with "no eggs," "reduced salt," "reduced calorie" or the like according to the conditional cooking instructions 58.

Also in the above cooking process, hygienic control is desirably carried out according to HACCP similarly to the preparation process. The CPU 200 extracts check items corresponding to cooking registered in the cooking master file 222 from the hygienic control database 224, and the printer 212 prints out a hygienic control checklist 48 corresponding to the cooking instructions 56 accordingly.

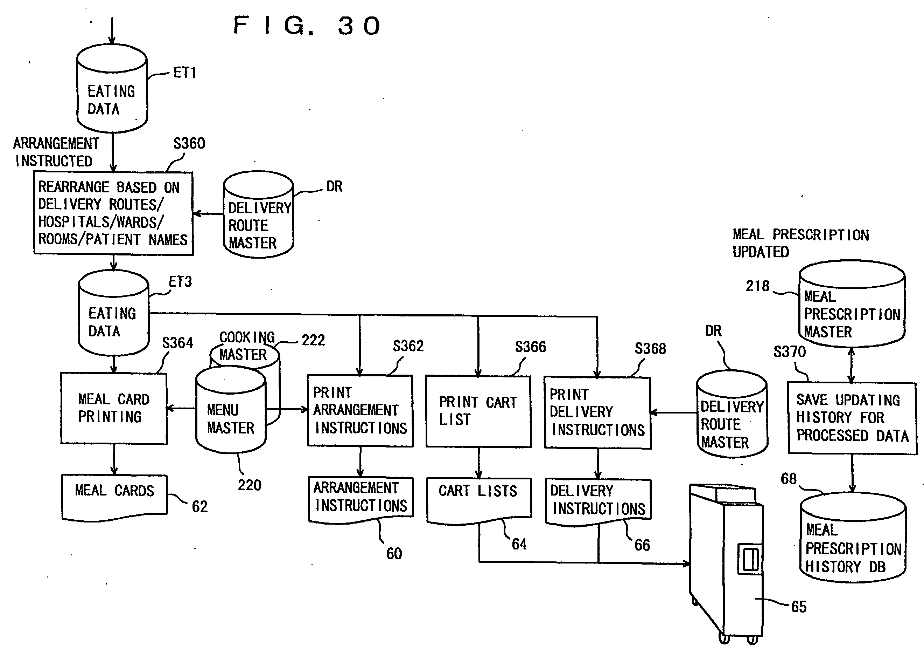

Referring to Fig. 30, the CPU 200 then outputs arrangement instructions. More specifically, the CPU 200 searches through the meal prescription mater 218 and once again extracts eating data ET1 on the input eating date and time (S26). Therefore, the same data as the eating data ET1 shown in Fig. 10 is once again obtained.

The CPU 200 rearranges the eating data ET1 into the order of delivery routes, hospitals, wards, rooms, and patients' names by referring to the delivery route master file DR (S360). Efficient routes for delivering meals depending upon the locations of hospitals that ordered the meals are pre-registered in the delivery route master file DR. When for example meals are delivered to "North hospital" and then to "South hospital," the CPU 200 selects eating data for "North hospital" and then eating data for "South hospital" from the eating data ET1. Then, the CPU selects data for the same ward, then for the same room, and then data for the next room. As a result of step S360, eating data ET3 is obtained.

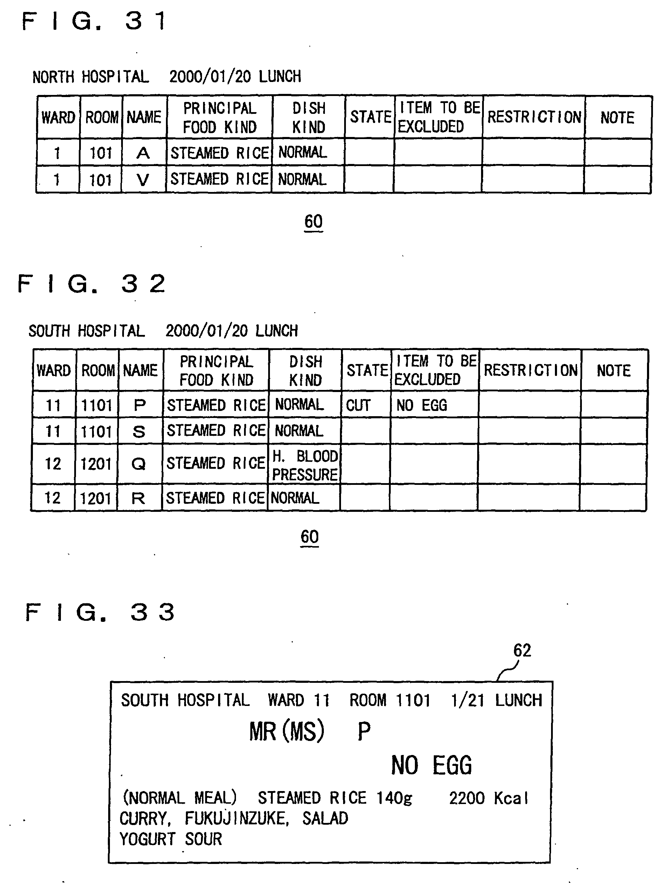

The printer 212 then prints out arrangement instructions 60 indicating the contents of meals for patients based on the eating data ET3 (S362). For example for the eating data shown in Fig. 10, the arrangement instructions 60 as shown in Fig. 31 are output for "North hospital," and the arrangement instructions 60 as shown in Fig. 32 are output for "South hospital."

In this step S362, the CPU 200 searches through the menu master file 220 for menus for date and time of interest, and the printer 212 prints a menu for each dish kind. The worker in charge of arrangement arranges meals for patients on trays (not shown) according to the arrangement instructions 60.

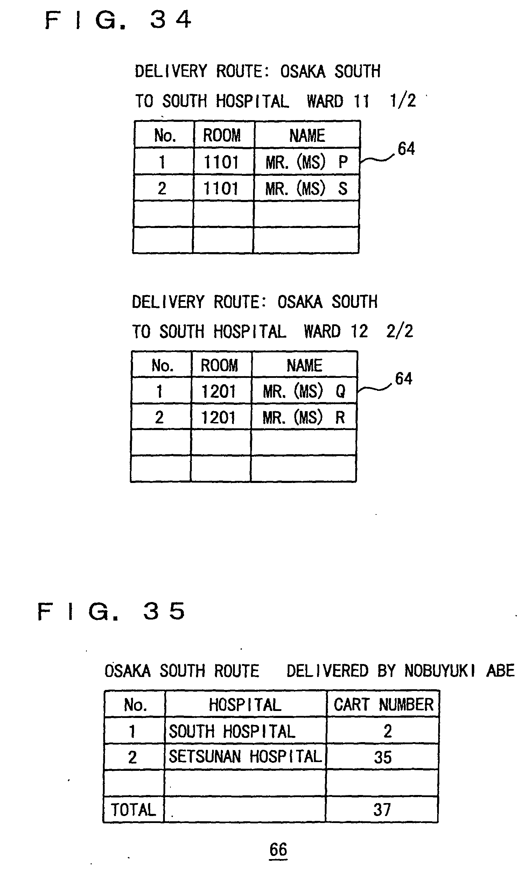

The printer 212 prints meal cards 62 in the same order as the arrangement instructions 60 (S364). For example for patient P shown in Fig. 32, the meal card 62 as shown in Fig. 33 is printed. The meal card 62 includes the name of the hospital, the ward number, the room number, the patient's name, and the contents of the meal. Therefore, each patient can confirm with the meal card 62 that the meal is surely appropriate for his/her conditions.

The printer 212 then prints a cart list 64 based on the eating data ET3 (S366). The cart list 64 indicates meals to be loaded onto each cook-chill cart 65.

In the example shown in Fig. 32, the cart list 64 as shown in Fig. 34 is output. The cook-chill cart can typically load about 20 trays, while in this example, patients P and S are in ward 11, South hospital, and patients Q and R are in ward 12, South hospital. Therefore, their meals are loaded separately into two cook-chill carts 65. The operator loads the meals arranged on the trays into the cook-chill carts 65 according to the cart list 64.

The cook-chill cart 65 has a timer that can be set to switch from the refrigeration mode to the heating mode at prescribed time. The timer can be used to set desired time to activate the heater, while a desired time period from setting of the timer to the heater activation may be set. The operator in charge of loading loads the meals arranged on the trays into the cook-chill carts 65 in the refrigeration mode, and sets the timer at the time.

The CPU 200 then searches through the delivery route master file DR, and the printer 212 prints out delivery instructions 66 based on the result of searching (S368). The delivery instructions 66 includes the order of delivering the cook-chill carts 65 to the hospitals, and the numbers of cook-chill carts 65 to be delivered to the hospitals, and for example what is shown in Fig. 35 is output. The truck driver in charge of delivery delivers the cook-chill carts 65 to the hospitals according to the delivery instructions 66.

Finally, the CPU 200 deletes the meal prescription data after the processing from the meal prescription master file 218, and stores the history in a meal prescription history database 68 (S370).

The hospitals 12 serve the delivered meals to the patients. About 30 minutes before the meals are served, the timer operates to switch the cook-chill carts 65 from the refrigeration mode to the heating mode, so the patients can have warm meals.

According to the embodiment, principal food portions of the same kind can be cooked in bulk at a time, while various different dishes can be cooked in bulk when their preparation and cooking methods are the same. The cooked meals can be delivered in bulk to each hospital. Therefore, when orders from a large number of hospitals for meals by out-of-hospital cooking are received, the meals can be efficiently cooked and accurately provided to the patients in the hospitals.

Note that according to the embodiment, the meal prescriptions are transmitted to the food service company from each hospital through the Internet 14. More specifically, the meal prescription data may be transmitted by FTP (File Transfer Protocol) to an FTP server from a personal computer or the like. A mobile terminal, a mobile telephone or the like may be used to transmit e-mail messages. Meanwhile, communication through web pages by ASP (Active Server Page), CGI (Common Gateway Interface) or the like, personal computer communication, communication by a private or public line, and other wire or wireless communication may be utilized.

When patients are allowed to select favorite menus from a plurality of menus, in other words, selective menus are employed, identification information such as a number indicating a selected menu is attached to meal prescription data, so that meals can be efficiently cooked and accurately supplied to the patients in the hospitals.

In the description of the embodiment, the process from cooking to delivery is mainly described, while in the process of ordering ingredients before cooking, eating data for a specified date may be extracted from the meal prescription master file 218, the number of meals for each menu may be calculated and the eating data may be transmitted to an existing ordering and purchase system. In this way, the number of meals to cook does not have to be once again calculated for input in the ordering and purchase system.

The cost must be calculated by multiplying unit prices defined for each hospital by contract and actually delivered meal numbers for billing. The information on the number to be delivered is transmitted to an existing sales management system, so that the number of actually delivered meals does not have to be once again input in the sales management system.

Furthermore, the actual number of delivered meals for each dish kind for each hospital derived from the meal prescription master file 218 is transmitted to an existing nutrition management system, so that a nutrition management notebook such as a daily total consumption table can automatically be produced.

According to the invention, the storage medium can be an FD, a DVD, a ROM, a RAM and a server for an Internet service provider in addition to the CD-ROMs 16, 18 as described above. The out-of-hospital cooking management program or the meal ordering program can be distributed through the Internet.

The disclosed embodiments are illustrative only in every sense and should not be construed as limitative. The scope of the present invention is defined by the scope of claims for patent rather the above description, and therefore all the variations within the scope of claims and meaning of equivalent are included.

## Claims

1. An out-of-hospital cooking management method using a computer, comprising the steps of:
registering a meal prescription, in response to orders from a plurality of hospitals, in a meal prescription master file, said meal prescription including information related to a patient, a hospital where the patient is hospitalized, and a meal for the patient;
searching through said meal prescription master file and outputting cooking instructions for dishes in bulk to be cooked by the same cooking method among a plurality of kinds of dishes;
searching through said meal prescription master file and outputting arrangement instructions for meals; and
searching through said meal prescription master file and outputting shipping instructions for meals in bulk to be delivered to the same hospital.

2. The out-of-hospital cooking management method according to claim 1, wherein
said step of outputting said cooking instructions comprises the step of outputting principal food cooking instructions for principal food portions in bulk to be cooked by the same method.

3. The out-of-hospital cooking management method according to claim 1, wherein
said step of outputting said cooking instructions comprises the step of outputting preparation instructions for accompanying dishes in bulk to be prepared by the same preparation method.

4. The out-of-hospital cooking management method according to claim 1, wherein
said step of outputting said cooking instructions comprises the step of outputting cooking instructions for accompanying dishes in bulk to be cooked by the same cooking method.

5. The out-of-hospital cooking management method according to any one of claims 1 to 4, further comprising the step of extracting meal prescriptions for the same eating date and time from said meal prescription master file and producing an eating data file.

6. The out-of-hospital cooking management method according to claim 5, wherein
said step of outputting said principal food cooking instructions comprises the step of searching through said eating data file and calculating the number of principal food portions of the same kind.

7. The out-of-hospital cooking management method according to claim 5, wherein
said step of outputting said preparation instructions comprises the steps of:
searching through said eating data file and calculating the number of accompanying dishes of the same kind;
searching through a menu master file that stores the contents of accompanying dishes and specifying the contents of each accompanying dish; and
searching through a cooking master file that stores preparation methods and presenting a preparation method for each said accompanying dish whose contents are specified.

8. The out-of-hospital cooking management method according to claim 5, wherein
said step of outputting said cooking instructions comprises the steps of:
searching through said eating data file and calculating the number of accompanying dishes of the same kind;
searching through a menu master file that stores the contents of accompanying dishes, and specifying the contents of each accompanying dish; and
searching through a cooking master file that stores cooking methods, and presenting a cooking method for each said accompanying dish whose contents are specified.

9. The out-of-hospital cooking management method according to claim 1, further comprising the step of printing a meal card having at least the name of a patient, wherein
said step of outputting said shipping instructions comprises the step of printing a cart list having at least the names of patients to receive a plurality of meals to be loaded on each cart.

10. The out-of-hospital cooking management method according to claim 9, wherein
said step of outputting said shipping instructions further comprises the step of printing delivery instructions having at least the name of a hospital to which said cart is to be delivered.

11. An out-of-hospital cooking management system using a computer, comprising:
a meal prescription master file for storing a meal prescription including information related to a patient, a hospital where the patient is hospitalized, and a meal for the patient;
meal prescription registering means for registering said meal prescription in said meal prescription master file in response to orders received from a plurality of hospitals;
cooking instruction means for searching through said meal prescription master file and outputting cooking instructions for dishes in bulk to be cooked by the same cooking method among a plurality of kinds of dishes;
arrangement instruction means for searching through said meal prescription master file and outputting arrangement instructions for meals; and
shipping instruction means for searching through said meal prescription master file and outputting shipping instructions for meals in bulk to be delivered to the same hospital.

12. The out-of-hospital cooking management system according to claim 11, wherein
said cooking instruction means comprises principal food cooking instruction means for outputting principal food cooking instructions for principal food portions in bulk to be cooked by the same cooking method.

13. The out-of-hospital cooking management system according to claim 11, wherein
said cooking instruction means comprises preparation instruction means for outputting preparation instructions for accompanying dishes in bulk to be prepared by the same preparation method.

14. The out-of-hospital cooking management system according to claim 11, further comprising cooking instruction means for outputting cooking instructions for accompanying dishes in bulk to be cooked by the same cooking method.

15. The out-of-hospital cooking management system according to any one of claims 11 to 14, further comprising means for extracting meal prescriptions for the same eating date and time from said meal prescription master file and producing an eating data file.

16. The out-of-hospital cooking management system according to claim 15, wherein
said principal food cooking instruction means comprises means for searching through said eating data file and calculating the number of principal food portions of the same kind.

17. The out-of-hospital cooking management system according to claim 15, wherein
said preparation instruction means comprises:
a menu master file that stores the contents of accompanying dishes;
a cooking master file that stores preparation methods;
means for searching through said eating data file and calculating the number of accompanying dishes of the same kind;
means for searching through said menu master file and specifying the contents of each accompanying dish; and
means for searching through said cooking master file and presenting a preparation method for each said accompanying dish whose contents are specified.

18. The out-of-hospital cooking management system according to claim 15, wherein
said cooking instruction means comprises:
a menu master file that stores the contents of accompanying dishes;
a cooking master file that stores cooking methods;
means for searching through said eating data file and calculating the number of accompanying dishes of the same kind;
means for searching through said menu master file and specifying the contents of each accompanying dish; and
means for searching through said cooking master file and presenting a cooking method for each said accompanying dish whose contents are specified.

19. The out-of-hospital cooking management system according to claim 11, further comprising means for printing a meal card having at least the name of a patient, wherein
said shipping instruction means comprises means for printing a cart list having at least the names of patients to receive a plurality of meals to be loaded on each cart.

20. The out-of-hospital cooking management system according to claim 19, wherein
said shipping instruction means further comprises means for printing delivery instructions including at least the name of a hospital to which said cart is to be delivered.

21. An out-of-hospital cooking management program product that enables a computer to execute the steps of:
registering a meal prescription, in response to orders from a plurality of hospitals, in a meal prescription master file, said meal prescription including information related to a patient, a hospital where the patient is hospitalized, and a meal for the patient;
searching through said meal prescription master file and outputting cooking instructions for dishes in bulk to be cooked by the same cooking method among a plurality of kinds of dishes;
searching through said meal prescription master file and outputting meal arrangement instructions; and
searching through said meal prescription master file and outputting shipping instructions for meals in bulk to be delivered to the same hospital.

22. The out-of-hospital cooking management program product according to claim 21, wherein
said step of outputting said cooking instructions comprises the step of outputting principal food cooking instructions for principal food portions in bulk to be cooked by the same method.

23. The out-of-hospital cooking management program product according to claim 21, wherein
said step of outputting said cooking instructions comprises the step of outputting preparation instructions for accompanying dishes in bulk to be prepared by the same preparation method.

24. The out-of-hospital cooking management program product according to claim 21, wherein
said step of outputting the cooking instructions comprises the step of outputting cooking instructions for accompanying dishes in bulk to be cooked by the same cooking method.

25. The out-of-hospital cooking management program product according to any one of claims 21 to 24, further comprising the step of extracting meal prescriptions for the same eating date and time from said meal prescription master file and producing an eating data file.

26. The out-of-hospital cooking management program product according to claim 25, wherein
said step of outputting said principal food cooking instructions comprises the step of searching through said eating data file and calculating the number of principal food portions of the same kind.

27. The out-of-hospital cooking management program product according to claim 25, wherein
said step of outputting said preparation instructions comprises the steps of:
searching through said eating data file and calculating the number of accompanying dishes of the same kind;
searching through a menu master file that stores the contents of accompanying dishes and specifying the contents of each accompanying dish; and
searching through a cooking master file that stores preparation methods and presenting a preparation method for each said accompanying dish whose contents are specified.

28. The out-of-hospital cooking management program product according to claim 25, wherein
said step of outputting said cooking instructions comprises the steps of:
searching through said eating data file and calculating the number of accompanying dishes of the same kind;
searching through a menu master file that stores the contents of accompanying dishes, and specifying the contents of each accompanying dish; and
searching through a cooking master file that stores cooking methods, and presenting a cooking method for each said accompanying dish whose contents are specified.

29. The out-of-hospital cooking management program product according to claim 21, further comprising the steps of printing a meal card having at least the name of a patient, wherein
said step of outputting said shipping instructions comprises the step of printing a cart list having at least the names of patients to receive a plurality of meals to be loaded on each cart.

30. The out-of-hospital cooking management program product according to claim 29, wherein
said step of outputting said shipping instructions further comprises the step of printing delivery instructions having at least the name of a hospital to which said cart is to be delivered.

31. A computer-readable storage medium storing the out-of-hospital cooking management program product according to any one of claims 21 to 30.

32. An out-of-hospital cooking method using a cart having a timer that can be set so that a refrigeration mode can be switched to a heating mode at prescribed time, comprising the steps of:
preparing ingredients for cooking a meal in response to an order received from a hospital;
cooking said prepared ingredients;
arranging said cooked meal on a tray;
loading said tray having said meal arranged thereon onto a cart in said refrigeration mode and setting said timer; and
delivering the cart loaded with said tray to said hospital.
